# FASCICULE DE BREVET EUROPEEN

(11) **EP 2 056 802 B1**
(45) Date de publication et mention de la délivrance du brevet: **13.03.2013**
(21) Numéro de dépôt: 07802689.5
(22) Date de dépôt: 17.08.2007
(51) Int. Cl.: A61K 31/05, A61K 31/192, A61K 31/194, A61K 31/225, A23L 1/30, A61K 31/7004, A61P 3/10, A61P 3/06, A61P 3/04, A61P 9/12

(54) **COMPOSITION ANTIDIABETIQUE CONTENANT DE L'ACIDE CHICORIQUE ET/OU L'UN DE SES MÉTABOLITES**
ANTI-DIABETES-ZUSAMMENSETZUNG MIT CHICOREE-SÄURE UND/ODER EINEM IHRER METABOLITEN
ANTI-DIABETES COMPOSITION CONTAINING CHICORIC ACID AND/OR ONE OF THE METABOLITES THEREOF

(30) Priorité: 18.08.2006 FR 0607382
(43) Date de publication de la demande: 13.05.2009
(73) Titulaire: Centre National de la Recherche Scientifique (CNRS), 75116 Paris (FR)
(72) Inventeur: ANDARY, Claude, F-34830 Clapiers (FR); RIBES, Gérard, F-34070 Montpellier (FR); TOUSCH, Didier, F-34090 Montpellier (FR); AZAY-MILHAU, Jacqueline, 34430 Saint Jean de Vedas (FR); LAJOIX, Anne-Dominique, 34090 Montpellier (FR)
(74) Mandataire: Regimbeau
(86) Numéro de dépôt international: PCT/EP2007/058580
(87) Numéro de publication internationale: WO 2008/022974

(56) Documents cités:
- EP-A- 1 559 421
- WO-A-00/15215
- WO-A-00/47045
- WO-A-02/064536
- WO-A-03/029183
- DE-A1- 1 949 822
- FR-A1- 2 638 967
- JP-A- 61 040 763
- US-A1- 2004 043 057
- US-A1- 2004 192 773
- US-A1- 2006 069 161
- NOMURA EISAKU ET AL: "Synthesis of amide compounds of ferulic acid, and their stimulatory effects on insulin secretion in vitro." BIOORGANIC AND MEDICINAL CHEMISTRY, vol. 11, no. 17, 15 août 2003 (2003-08-15), pages 3807-3813, XP002425367 ISSN: 0968-0896
- OHNISHI M ET AL: "Antioxidant activity and hypoglycemic effect of ferulic acid in STZ-induced diabetic mice and KK-Ay mice" BIOFACTORS, vol. 21, no. 1-4, 2004, pages 315-319, XP008076266 ISSN: 0951-6433
- DATABASE WPI 10 novembre 2005 (2005-11-10), Thomson Scientific, London, GB; Class 057,page 8, AN 2005-763566 XP002425373 TAGASHIRA EIJIRO ET AL.: "Cardiovascular disease medicine and health food" & JP 2005 314435 A (TAGASHIRA EIJIRO) 10 novembre 2005 (2005-11-10)
- DATABASE WPI 25 septembre 2002 (2002-09-25), Thomson Scientific, London, GB; Class 033,page 3, AN 2003-345667 XP002425374 CHIKUNO T ET AL.: "Hypoglycemic agent" & JP 2002 275058 A (MORISHITA H ET AL.) 25 septembre 2002 (2002-09-25)
- JUNG UN JU ET AL: "Antihyperglycemic and antioxidant properties of caffeic acid in db/db mice" JOURNAL OF PHARMACOLOGY AND EXPERIMENTAL THERAPEUTICS, vol. 318, no. 2, août 2006 (2006-08), pages 476-483 URL, XP008076265 ISSN: 0022-3565
- HSU FENG-LIN ET AL: "Caffeic acid as active principle from the fruit of Xanthium strumarium to lower plasma glucose in diabetic rats" PLANTA MEDICA, vol. 66, no. 3, avril 2000 (2000-04), pages 228-230, XP008076263 ISSN: 0032-0943
- DATABASE WPI 2 août 1986 (1986-08-02), Thomson Scientific, London, GB; Class 863,page 7, AN 1986-242356 XP002425375 FUWA T. ET AL.: "Antidiabetic" & JP 61 171418 A (WAKUNAGA SEIYAKU KK) 2 août 1986 (1986-08-02)
- DATABASE WPI 22 avril 2003 (2003-04-22), Thomson Scientific, London, GB; Class 036,page 3, AN 2003-666734 XP002425376 KANO H. ET AL.: "Food composition having inhibitory action on postprandial elevation of blood glucose." & JP 2003 116486 A (MEIJI MILK PROD CO LTD) 22 avril 2003 (2003-04-22)
- MATSUI T ET AL: "Anti-hyperglycemic potential of natural products" MINI-REVIEWS IN MEDICINAL CHEMISTRY 2006 NETHERLANDS, vol. 6, no. 3, 2006, pages 349-356, XP008076264 ISSN: 1389-5575
- STADLER, RICHARD H. ET AL: "The inhibitory effects of coffee on radical-mediated oxidation and mutagenicity" MUTATION RESEARCH , 308(2), 177-90 CODEN: MUREAV; ISSN: 0027-5107, 1994, XP002425368
- BAILLY FABRICE ET AL: "Anti-HIV activities of natural antioxidant caffeic acid derivatives: Toward an antiviral supplementation diet" CURRENT MEDICINAL CHEMISTRY, vol. 12, no. 15, 2005, pages 1811-1818, XP008076705 ISSN: 0929-8673
- SRI BALASUBASHINI M ET AL: "Protective effects of ferulic acid on hyperlipidemic diabetic rats." ACTA DIABETOLOGICA, vol. 40, no. 3, septembre 2003 (2003-09), pages 118-122, XP002478246 ISSN: 0940-5429
- KIM HAE KYUNG ET AL: "Lipid-lowering efficacy of hesperetin metabolites in high-cholesterol fed rats." CLINICA CHIMICA ACTA, vol. 327, no. 1-2, janvier 2003 (2003-01), pages 129-137, XP002478247 ISSN: 0009-8981
- TOUSCH D ET AL: "Chicoric acid, a new compound able to enhance insulin release and glucose uptake", BIOCHEMICAL AND BIOPHYSICAL RESEARCH COMMUNICATIONS, ACADEMIC PRESS INC. ORLANDO, FL, US, vol. 377, no. 1, 5 December 2008 (2008-12-05), pages 131-135, XP025587436, ISSN: 0006-291X, DOI: 10.1016/J.BBRC.2008.09.088 [retrieved on 2008-10-01]
- ANONYMOUS: 'Diabetes health center Risk factors for diabetes', [en ligne] 11 Août 2011, WebMD Extrait de l'Internet: <URL:http://diabetes.webmd.com/risk-factors -for-diabetes> [extrait le 2011-08-11]

## Description

La présente invention se rapporte à une composition pour utilisation dans la prévention ou le traitement cree un sujet de l'insulino-résistance ou de l'hypo-insulémie et/ou des pathologies associés telles que définies dans les revendications caractérisée en ce qu'elle comprend de l'acide chiconique.

Il est reconnu que le diabète touche actuellement plusieurs millions d'individus dans le monde. Le nombre de personnes diabétiques atteindrait 300 millions en 2025 (1). Par conséquent le diabète constitue un problème majeur de santé publique.

On distingue actuellement plusieurs sortes de diabète-sucré :
- le diabète de type 1, insulino-dépendant,
- le diabète de type 2, non insulino-dépendant

Le diabète de type 2 (diabète non insulino-dépendant) est de loin le plus fréquent (environ 90% des cas) Autrefois appelé diabète de l'âge mûr, ce type de diabète survient principalement chez l'adulte de plus de 40 ans présentant, dans 80% des cas, une obésité ou du moins un excès pondéral. Au début de la maladie, la production d'insuline par le pancréas peut être anormale : soit excessive, soit diminuée.

Chez les patients atteints du diabète de type 2, deux altérations métaboliques sont principalement la cause d'une élévation de la glycémie (hyperglycémie) :
1- Un déficit insulinique (donc pancréatique) à la réponse endocrine au glucose.
2- Un déficit de l'action de l'insuline sur les tissus périphériques (insulino-résistance) : muscles et tissus adipeux principalement (effet extrapancréatique).

Cette altération de la réponse insulinique au glucose peut donc être due à deux causes suivant les stades de la maladie:
soit une trop faible sécrétion d'insuline sous l'effet du glucose, soit une hypersécrétion d'insuline afin de compenser la diminution de son action sur les tissus cibles.

Le diabète de type 2 est traité au départ par des mesures hygiéno-diététiques et notamment par la perte d'excès de poids. L'étape suivante est le traitement pharmacologique par des antidiabétiques oraux : des substances qui agissent en stimulant la sécrétion d'insuline (insulino-stimulantes) afin de compenser le déficit : ce sont principalement les sulfonylurées et les glinides (qui agissent principalement sur le canal potassique, ATP dépendant).

L'autre alternative thérapeutique est d'agir pour améliorer l'action de l'insuline (insulino-sensibilisateur). Deux groupes de substances sont reconnues : les biguanides (la metformine) et les glitazones (Rosi-, Tro-, Pio-glitazones).

Si tout ceci échoue, une insulinothérapie ponctuellement substitutive peut s'avérer nécessaire pour maintenir une glycémie normale.

Les substances principales capables de stimuler la sécrétion d'insuline (insulino-stimulant) sont les sulfamides hypoglycémiants (sulfonylurées) ou les substances apparentées (glinides). Ces substances qui agissent principalement sur le canal potassique ATP dépendant de la cellule β pancréatique présentent toutefois un certain nombre d'inconvénients.

Ainsi, suivant la posologie et la susceptibilité des personnes il peut en résulter notamment chez les personnes âgées, des hypoglycémies par excès de sécrétion d'insuline. Ceci peut conduire à un coma hypopglycémique.

Pour remédier à ces inconvénients, les chercheurs se sont orientés vers d'autres alternatives pharmacologiques de substances insulino-stimulantes agissant sur d'autres cibles que le canal potassique ATP dépendant, c'est-à-dire modulatrices de la sécrétion d'insuline en fonction du taux de glucose circulant, évitant ainsi les complications telles que les hypoglycémies post-traitement. En particulier, la recherche de nouvelles molécules pharmacologiques actives s'oriente de plus en plus vers les substances naturelles, extraites de plantes (2).

Ainsi, cette recherche de composés naturels a permis de mettre en évidence des composés phénoliques dérivés de l'acide caféique comme l'acide chlorogénique purifié à partir de nombreuses plantes.

Cet acide chlorogénique agit au niveau hépatique donc extrapancréatique est réputé agir au niveau de la néoglucogénèse hépatique. En effet, l'acide chlorogénique possède une activité inhibitrice de la glucose-6-phosphatase (3), essentiellement hépatique, et permet donc une meilleure captation hépatique du glucose puis une meilleure tolérance au glucose. L'acide chlorogénique n'a pas d'action directe sur le pancréas endocrine et ne possède donc pas une action insulino-stimulante mais possède une action insulino-sensibilisatrices permettant de lutter contre l'insulino-résistance des tissus périphériques.

L'insulino-résistance est la résistance de l'organisme aux effets biologiques hypoglycémiants de l'insuline. De ce fait, ces composés insulino sensibilisant sont utilisés pour des patients souffrant d'une telle résistance à l'insuline.

Nomura et al. divulguent dans Bioorg. Med. Chem., 11(17), 3807-3813 (2003) que l'acide férulique réduit le taux de glucose chez les souris diabétiques (diabète STZ-induit) et augmente la sécrétion d'insuline dans des cellules pancréatiques RIN-5F issues de pancréas de rat.

Jung et al. décrivent dans J. Pharm. Exper. Ther., 318(2), 476-483 (2006) la réduction du taux de glucose sanguin chez les rats après administration de l'acide caféique. L'acide caféique a un double mécanisme d'action: il augmente la libération d'insuline du pancréas et améliore l'insulino-résistance dans le foie et dans les tissus adipeux.

JP-A-61 171 418 divulgue un agent antidiabétique avec comme principe actif un acide carboxylique, comme l'acide L-, D-, DL- ou méso-tartrique. L'agent ne provoque pas d'hypoglycémie et n'agit que lorsque la concentration de glucose sanguine est élevée.

US-A-2004/192773 décrit l'utilisation de dérivés ou de pro-drogues de l'acide férulique, comme l'acide dicafféoyl tartrique pour le traitement de l'hypertension.

Cependant aucune substance phénolique naturelle ou de synthèse dérivée de l'acide caféique n'a été décrite aujourd'hui ayant une activité insulino-stimulante de la sécrétion d'insuline par les cellules β du pancréas dependant de la concentration en glucose dans le sang du sujet à traiter.

Les inventeurs de la présente invention ont extrait de plantes, et purifié, l'acide chicorique (acide dicaféoyl-tartrique) dont la formule est la suivante:

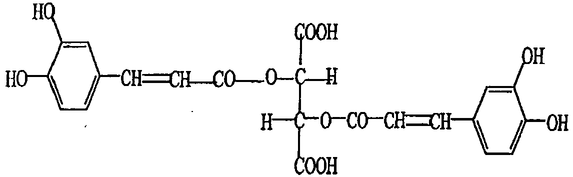

L'acide chicorique qui est un diester caféique de l'acide tartrique, entre dans la catégorie des combinaisons naturelles des acides hydroxy-cinnamiques qui possèdent de nombreuses vertus médicinales telles que des activités comme anti-oxydante, anti-virale ou anti-cancéreuse (4,5,6). Ces combinaisons purifiées à l'origine dans le café particulièrement riche en dérivés cinnamiques ont été très largement étudiées. L'acide chicorique peut se trouver sous forme de trois isomères selon l'isomérie de l'acide tartrique: l'acide dicaféoyl-(2S, 3S)-(+)- tartrique, l'acide dicaféoyl-(2R,3R)-(-) tartrique et l'acide dicaféoyl-méso-tartrique (7).

L'acide chicorique est présent chez de nombreuses espèces végétales appartenant plus particulièrement aux familles suivantes: Fabaceae, Asteraceae, Lamiacea, Equisetaceae ou Potamogetonaceae ou autres, telles que par exemple la chicorée (*Cichorium inllibus*), l'échinacée (Echinacea purpurea), l'arachide (*Arachis hypogaea*), la prèle (*Equisetum arvense*), le pissenlit (*Taraxacum officinalis*), la laitue (*Lactuca sativa*) ou la posidonie (Posidonia oceanica). C'est souvent le composé majoritaire de toutes les espèces et familles de plantes précitées qui peuvent donc servir à l'extraction et la purification de cette substance.

L'acide chicorique peut donc être isolé et purifié à partir de plantes telles que décrites ci-dessus. Un exemple de procédé de production et isolement d'acide chicorique naturel à partir de Chicorée sauvage (*Cichorium pumilum,* C. *eu-endivia* ou *C*. *intybus*) est donné ci après.

Après séchage et broyage la poudre de chicorée est extraite par de l'eau à ébullition pendant 20 minutes. Après refroidissement l'extrait est concentré sous vide entre 30°C et 40°C. Ce nouvel extrait est déposé sur une colonne adsorbante (type Duolite S 761 ou XAD 761 ou autre adsorbant de même type) qui aura été lavée et activée au préalable. Une première élution à l'eau légèrement acidifiée (pH 4) permet de séparer les sucres, les acides organiques et autres petites molécules non aromatiques, tout en laissant adsorbées les molécules organiques aromatiques à fonctions phénoliques ou carbonylées. Le décrochage de ces dernières molécules se fait avec un mélange: éthanol-eau (80: 20), Les extraits obtenus, enrichis en acide chicorique, sont concentrés et lyophilisés.

La purification de cette molécule, à partir de la poudre lyophilisée, se fait par chromatographie liquide sur colonne de cellulose à moyenne pression (CLMP). Le solvant d'élution est de l'acide acétique à 0,10 ou 0,15 % dans l'eau. Les fractions les plus riches en acide chicorique sont regroupées, concentrées et cet extrait est purifié par une chromatographie sur colonne de Fractogel TSK RW 40 (F). L'élution est alors réalisée par de l'eau suivie d'un gradient d'éthanol dans l'eau. Les fractions contenant l'acide chicorique pur sont lyophilisées.

D'autres procédés d'obtention d'acide chicorique sont décrits dans « Scarpati et al. (8) : « Chicoric Acid (Dicaffeyltartaric acid) : Its isolation from Chicory and synthesis » Tetrahedron, 1958, Vol. 4, pp. 43-48 pour l'obtention d'acide chicorique naturel, ou dans la demande WO03/029183 pour l'obtention d'acide chicorique synthétique.

De manière surprenante et inattendue, les inventeurs ont montré que l'acide chicorique extrait de plantes présente des propriétés insulino-stimulantes, non canal potassique, ATP dépendant, et on obtenu des résultats significatifs dans la prévention et le traitement du diabète, en particulier non insulino-dépendant. En effet, l'effet insulino-stimulant de l'acide chicorique a été décrit par des expériences *in vitro* sur la lignée INS1E, sur les îlots de Langerhans isolés de pancréas de rat et des expériences *in vivo* chez des rats normaux. Les résultats obtenus sur la lignée INS1E montrent que l'effet de l'acide chicorique atteint très rapidement un maximum de stimulation, inférieur aux valeurs de stimulation obtenue en utilisant un médicament de type sulfonylurée tel que le tolbutamide connu pour leur action sur le canal potassique ATP dépendant de la cellule β pancréatique. Ce résultat suggère que l'acide chicorique n'agit probablement pas sur ce canal. De plus, des expériences récentes des inventeurs sur des îlots de Langerhans isolés de pancréas de rat ont permis de montrer que l'effet insulino-stimulant de l'acide chicorique est dépendant de la concentration de glucose. L'acide chicorique peut donc être une bonne alternative innovante dans la prévention ou le traitement de l'hypoinsulinémie, en particulier du diabète car il est capable de stimuler la sécrétion d'insuline sans pour autant provoquer d'hypoglycémie chez le patient comme cela arrive parfois chez les personnes traitées par des sulfonylurées ce qui peut devenir un danger notamment chez les personnes âgées.

Par ailleurs, rappelons que l'un des principaux facteurs de risque dans le déclenchement d'un diabète de type 2 est la prise excessive de poids souvent liée à la sédentarité et/ou une alimentation mal équilibrée. Ce dernier point devient un nouveau fait sociétal inquiétant pour les pays industrialisés. L'obésité concourre en effet au développement d'une insulino-résistance c'est-à-dire lorsque l'action de l'insuline sur les tissus cibles extra-pancréatiques (muscles, foie) est diminuée, le captage du glucose par ces derniers est fortement altéré. L'insulino-résistance est très souvent suivie par une hyperinsulinémie compensatoire. Ceci résulte d'une incapacité relative à recruter à la membrane plasmique des cellules le transporteur du glucose, essentiellement GLUT4 pour les tissus musculaires et adipeux. Cette relation causale entre obésité et diabète de type 2 a pris la dimension d'un phénomène majeur sur le plan de la santé publique. Un faisceau de preuve lie l'hyperinsulinémie au risque d'accidents cardio-vasculaires et a permis de dresser le tableau du syndrome métabolique ou syndrome X de Reaven (insulino-résistance, hyperinsulinémie, hypertriglycéridémie, hypertension artérielle) avec les risques morbides qui lui sont associés.

En plus des propriétés insulino-stimulantes de l'acide chicorique, les inventeurs ont pu démontrer l'effet stimulant de l'acide chicorique sur le captage de glucose à une dose stimulante d'insuline, en particulier sur des tissus musculaires (principaux tissus de captage de glucose dans l'organisme) et donc l'effet insulino-sensibilisateur de l'acide chicorique.

L'acide chicorique peut donc être une bonne alternative innovante dans la prévention et le traitement de l'insulino-résistance.

Ainsi, la présente invention décrit une composition insulino-stimulante caractérisée en ce qu'elle comprend, en tant que substance active au moins de l'acide chicorique, telle que definie dans les revendications.

De manière avantageuse, l'acide chicorique est sous la forme d'un des isomères acide dicaféoyl (+), (-) ou mésotartrique.

Les métabolites de l'acide chicorique sont l'acide monocafeoyl-tartrique, l'acide caféique, l'acide tartrique, l'acide férulique, l'acide dihydrocaféique, l'acide m-hydroxyphénylpropionique, l'acide dihydroxyphenylacetique, le 4-vinylcatechol, ou le 4-ethylcatechol.

La présente description divulgue l'utilisation de l'acide chicorique et/ou d'au moins l'un de ses métabolites choisi parmi l'acide monocafeoyl-tartrique et/ ou l'acide m-hydroxyphenylpropionique, et/ou le 4-vinylcathecol et/ou le 4 ethylcatechol pour la fabrication d'une composition destinée à la prévention ou au traitement chez un sujet de l'insulino-résistance ou de l'hypo-insulinémie et/ou des pathologies associées.

De manière préférée, l'utilisation selon la présente invention est caractérisée en ce que la composition est insulino-stimulante et qu'elle est destinée à la prévention ou au traitement de l'hypo-insulinémie.

Par « hypoinsulinémie » on entend désigner selon la présente invention un déficit insulinique chez le sujet atteint, c'est-à-dire un taux d'insuline dans le sang du sujet anormalement bas. L'hypoinsulinémie peut provoquer une hyperglycémie chez le sujet atteint.

Les pathologies liées à l'insulino-résistance ou à l'hypoinsulinémie selon la présente invention sont choisies dans un groupe comprenant le diabète, les dyslipidémies, en particulier les hyperlipidémies et hypertriglicéridémie, le syndrome métabolique et l'obésité.

Par « syndrome métabolique » on entend désigner selon la présente invention la pathologie également appelée syndrome X ou syndrome X de Reaven.

Le syndrome métabolique est une pathologie définie par un ensemble de facteurs de risques incluant les dyslipidémies (taux faible deHDL-c, taux élevé de triglycérides), l'augmentation de la circonférence abdominale/obésité, mais aussi la résistance à l'insuline (hyperglycémie à jeun), et l'hypertension artérielle. Ce syndrome affecte plusieurs millions de personnes au monde, les exposant à un plus grand risque de développer un diabète avec ses complications d'insuffisance rénale et de rétinopathie, ou de provoquer une maladie cardiovasculaire telle que coronaropathie, insuffisance coronaire, infarctus du myocarde, angor, athérosclérose, artériosclérose, accident vasculaire cérébral, thromboses, athérothromboses ou glaucome, ou encore une maladie hépatique telle que stéatose, stéatohépatite non alcoolique, ou dégénérescence graisseuse non alcoolique du foie.

La prévention et le traitement du syndrome métabolique de patients à risque peuvent contribuer à diminuer l'apparition de maladies cardiovasculaires et de diabète de type 2 ou encore de maladies hépatiques.
La définition du syndrome métabolique n'est pas mondialement unifiée, celle donnée par le National Cholesterol Education Program (NCEP, USA), dans le cadre d'un groupe d'experts ATP III (de l'anglais Adult Treatment Panel III), et retenue pour la présente invention, retient les critères listés dans le tableau 1 suivant. Les patients ont un syndrome métabolique lorsqu'ils remplissent au moins 3 des 5 critères indiqués : augmentation de la circonférence abdominale, obésité, dyslipidémie, hypertension artérielle, hyperglycémie.

**Tableau 1**

| | **ATP III** |
|---|---|
| **Circonférence abdominale** | **Tour de taille :** |
| | Hommes > 102 cm |
| | Femmes > 88 cm |
| **Lipides** | **Triglycérides (TG) :** |
| | ≥ 150 mg/dl |
| | **HDL-c** (de l'anglais Highe Density Lipoprotein cholesterol) |
| | Hommes > 40 mg/dl |
| | Femmes > 50 mg/dl |
| **Pression sanguine** | ≥ 130/85 mm Hg |
| **Glycémie à jeun** | ≥_{:} 110 mg/dl |

La dyslipidémie est définie par une élévation des triglycérides, du LDL-c (de l'anglais Low Density Lipoprotein Cholesterol), par une concentration basse de HDL-c (de l'anglais High Density Lipoprotein Cholesterol), par l'augmentation du rapport cholestérol total/HDL-c, par la présence de particules de petites tailles de LDL. Cette dyslipidémie, souvent présente chez le sujet obèse, est également reconnue comme ayant un profil athérogénique, c'est à dire qui majore le risque de maladie athéromateuse.
De manière préférée, l'invention concerne le traitement ou la prévention du diabète en particulier le diabète insulino-dépendant ou le diabète non insulino-dépendant et encore plus préférentiellement le diabète non insulino-dependant.
De plus, comme indiqué précédemment, les inventeurs ont pu montrer que l'effet insulino-stimulant de l'acide chicorique est dépendant de la concentration de glucose. Ainsi, l'activité insulino-stimulante de la composition selon la présente invention est dépendante de la concentration du glucose dans le sang du sujet.
De manière préférée, la composition selon la présente invention est insulino-stimulante chez un sujet présentant une concentration sanguine en glucose supérieur à 1g/l.
De manière avantageuse, la composition selon la présente invention est insulino-sensibilisatrice.

L'effet insulino-sensibilisateur désigne selon la présente invention les propriétés de l'acide chicorique et de ses métabolites à prévenir ou traiter l'insulino-résistance, notamment en activant des récepteurs nucléaires à l'insulines au niveau des cellules adipeuses et musculaires. De tels récepteurs sont notamment les PPARs (Peroxisome Proliferator Activated Receptors).
Plusieurs mécanismes seraient impliqués dans cette action insulino-sensibilisatrice dont, notamment, une diminution de diverses molécules impliquées dans l'insulino-résistance comme les acides gras libres, la leptine et le TnF alpha, ces molécules s'opposant à l'action de l'insuline sur les cellules.

De manière préférée selon l'invention l'acide chicorique est d'origine naturelle ou synthétique.

De manière encore plus préférée selon l'invention, l'acide chicorique est d'origine végétale. Avantageusement, l'acide chicorique est obtenu à partir de plantes appartenant à la famille des Asteraceae, des Lamiaceae, des Fabaceae, d'Equisetaceae ou de Potamogetonaceae avantageusement les chicorées sauvages (ex : Cichorium intybus) ou cultivées (ex : le chicon ou endive).

Par Asteraceae, on entend selon la présente invention la famille des Asteraceae (ou Astéracées ou Composées) qui est une famille de plantes dicotylédones. Ce sont essentiellement des plantes herbacées même s'il peut exister des arbres, des arbustes ou des lianes dans cette famille.

De manière préférée, les genres préférés dans cette famille pour l'obtention d'acide chicorique selon la présente invention sont :
- Lactuca, les laitues.
- Cichorium, les chicorées dont le chicon (ou endive).
- Cynara, l'artichaut.
- Taraxacum, le pissenlit.
- Tragopogon, le salsifis.
- Echinacea, l'échinacée.
- Lapsana.

De manière préférée, les genres préférés dans cette famille pour l'obtention d'acide chicorique selon la présente invention sont :

Par Lamiaceae on entend selon la présente invention la famille des Lamiaceae (ou Lamiacées ou Labiées) qui est une famille de plantes dicotylédones,

De manière préférée, les genres préférés dans cette famille pour l'obtention d'acide chicorique selon la présente invention sont :
- Lavendula, la lavande
- Orthosiphon aristus, l'orthosifon
- Scutelleria, la scutellaire
- Teucrium

Par Fabaceae, on entend selon la présente invention la famille des Fabaceae (ou fabacées ou légumineuses) qui est une famille de plantes dicotylédones. Ce sont des plantes herbacées, des arbustes, des arbres ou des lianes.

De manière préférée, les genres préférés dans cette famille pour l'obtention d'acide chicorique selon la présente invention sont :
- les Papilionoidées ou Faboideae;
- les Caesalpinioidées;
- les Mimosoidées.

De manière préférée, les genres préférés dans cette famille pour l'obtention d'acide chicorique selon la présente invention sont :
- *Arachis hypogea,* l'arachide

Par Potamogetonacea, on entend selon la présente invention la famille des Potamogetonacea qui est une famille de plantes monocotylédones aquatiques.

De manière préférée, le genre préféré dans cette famille pour l'obtention d'acide chicorique selon la présente invention sont :
- *Posidonia,* la posidonie

Par Equisetaceae, on entend selon la présente invention la famille des Equisetaceae qui est une famille de plantes Ptéridophytes. De manière préféré, le genre préféré dans cette famille pour l'obtention de l'acide chicorique selon la présente invention sont :
- *Equisetum arvense,* la prêle

La présente invention décrit également l'utilisation de l'acide chicorique pour la préparation d'une composition destinée à stimuler la sécrétion d'insuline, chez un sujet diabétique.

La stimulation de la sécrétion d'insuline, entraîne une amélioration de la tolérance au glucose.

De ce fait, la présente demande décrit également l'utilisation de ladite composition pour améliorer la tolérance au glucose.

De plus, la stimulation de la sécrétion d'insuline ainsi que l'amélioration de la tolérance au glucose permet entre autre de prévenir ou traiter le diabète, en particulier le diabète non insulino dépendant.

Selon la présente invention, l'acide chicorique utilisé pour la préparation de la composition pour stimuler la sécrétion d'insuline et améliorer la tolérance au glucose pour prévenir et/ou traiter le diabète, est sous la forme d'un des isomères acide dicaféoyl (-), (+) ou mésotartrique.

La présente invention décrit que l'acide chicorique est utilisé pour la préparation de la composition pour stimuler la sécrétion d'insuline, améliorer la tolérance au glucose pour prévenir et/ou traiter le diabète est d'origine naturelle ou synthétique.

La présente invention décrit également que l'acide chicorique est utilisé pour la préparation de la composition pour stimuler la sécrétion d'insuline et améliorer la tolérance au glucose et/ou prévenir ou traiter le diabète est d'origine végétale ou de synthèse. Avantageusement, l'acide chicorique est obtenu à partir d'Asteraceae, de Lamiaceae, de Fabaceae, d'Equisetaceae ou de Potamogetonaceae, avantageusement les chicorées sauvages (ex : Cicorium intybus) ou cultivée.

De manière préférée, l'utilisation de la composition selon l'invention comprend l'administration au sujet de 5 à 30mg/Kg d'acide chicorique par prise.

La présente description divulgue une composition alimentaire caractérisée en ce qu'elle comprend au moins de l'acide chicorique et/ou l'un de ses métabolites.

La présente description décrit que l'acide chicorique et/ou l'un de ses métabolites de la composition alimentaire est d'origine naturelle ou synthétique.

La présente description décrit que l'acide chicorique et/ou l'un de ses métabolites de la composition alimentaire est d'origine végétale. Avantageusement, l'acide chicorique et/ou l'un de ses métabolites de la composition alimentaire est obtenu à partir d'Asteraceae, de Lamiaceae ou de Fabaceae, avantageusement les chicorées sauvages ou cultivées.

La composition alimentaire peut se présenter ous la forme d'une composition alimentaire caractérisée en ce qu'elle comprend au moins de l'acide chicorique et/ou au moins l'un de ses métabolites choisi parmi l'acide monocafeoyl-tartrique et/ ou l'acide m-hydroxyphenylpropionique, et/ou le 4-vinylcathecol et/ou le 4 ethylcatechol et en ce qu'il s'agit d'un complément nutritionnel. ,

Par complément nutritionnel on entend selon la présente description, et reprenant la définition de la directive 2002/46/CE du Parlement européen et du Conseil du 10 juin 2002, une denrée alimentaire dont le but est de compléter le régime alimentaire normal et qui constitue une source concentrée de nutriments ou d'autres substances ayant un effet nutritionnel ou physiologique seuls ou combinés, commercialisés sous forme de doses, à savoir les formes de présentation telles que les gélules, les pastilles, les comprimés, les pilules et autres formes similaires, ainsi que les sachets de poudre, les ampoules de liquide, les flacons munis d'un compte-gouttes et les autres formes analogues de préparations liquides ou en poudre destinées à être prises en unités mesurées de faible quantité. Il est à noter que la présente description, comprend également la définition américaine du complément nutritionnel couvrant « tout produit destiné à supplémenter une alimentation et qui contient un ou plusieurs ingrédients alimentaires spécifiés (vitamines, tisanes, plantes, acides aminés, un concentré, un extrait, un métabolite ou toute combinaison de ces derniers ingrédients) de façon à en augmenter la consommation quotidienne».

La composition alimentaire peut être sous la forme d'un nutraceutique.

Le terme « nutraceutique » fait référence à l'ingrédient actif présent à l'état naturel dans un aliment qui procure un effet bénéfique pour la santé. Par exemple, l'aillicine est une substance naturellement présente dans l'ail qui a un effet antioxydant protecteur contre les maladies cardiovasculaires et le cancer. L'aillicine est donc un nutraceutique, et ce, aussi bien lorsqu'elle est à l'état naturel que lorsqu'elle est commercialisée sous forme de poudre ou de comprimé. Le comprimé de brocoli concentré est un autre exemple de nutraceutique.

La composition alimentaire peut être sous la forme d'une poudre, d'une capsule, d'un comprimé, d'une solution, d'un concentré, d'un sirop, d'une suspension, ou d'une dispersion. De manière préférée, la composition alimentaire est sous la forme de comprimés, poudre, capsules, pilules, boisson.

De manière avantageuse, la composition alimentaire est ajoutée à un produit alimentaire.

De manière avantageuse, la composition alimentaire est ajoutée lors de la préparation du produit alimentaire, ou juste avant consommation du produit alimentaire. Par exemple, la composition alimentaire se présente sous la forme de poudre à saupoudrer sur un aliment prêt à consommer.

De manière préférée, la composition alimentaire comprend 200 à 600 mg/L d'acide chicorique et/ou au moins de 200 à 600 mg de l'un de ses métabolites, en particulier d'acide monocafeoyl-tartrique et/ ou d'acide m-hydroxyphenylpropionique, et/ou de 4-vinylcathecol et/ou de 4 ethylcatechol, par prise et pour un individu de 60 Kg.

De manière avantageuse la composition alimentaire est caractérisée en ce qu'elle comprend du glucose.

La présente description décrit également l'utilisation d'une composition alimentaire telle que décrite ci-dessus pour la prévention du diabète, avantageusement le diabète non insulino dépendant, et/ou l'amélioration de la tolérance au glucose.

L'utilisation non thérapeutique de la composition alimentaire pour la prévention du diabète, de l'obésité, du syndrome métabolique, de l'insulino-résistance liée au vieillissement et/ou l'amélioration de la tolérance au glucose, est aussi divulguée.

De manière avantageuse, la composition alimentaire comprend du glucose et est utilisé, de manière non thérapeutique, comme produit alimentaire énergétique. Il s'agit par exemple d'une boisson énergétique.

La présence combinée d'acide chicorique et/ou d'au moins un de ses métabolites en particulier l'acide monocafeoyl-tartrique et/ ou l'acide m-hydroxyphenylpropionique, et/ou le 4-vinylcathecol et/ou le 4 ethylcatechol, avec du glucose permet d'améliorer la disponibilité dudit glucose à l'organisme du consommateur, en particulier à ses muscles.

### Légende des figures

**Figure 1** **:** Démonstration de l'effet direct insulino-stimulant de l'acide chicorique (SAT2) sur la cellule β isolée de type INS 1E
**Figure 2** : Effet insulino-stimulant de l'acide chicorique (SAT2) sur des îlots de Langerhans isolés de rat incubés.
**Figure 3** : Effet de l'injection intrapéritonéale (IP) d'acide chicorique (SAT2) lors d'une épreuve d'hyperglycémie provoquée (1g/kg IP) chez le rat normal éveillé.
   A- Cinétiques de l'insulinémie après l'injection.
   B- Aires sous la courbe (ASC) de l'insulinémie lors des 30 premières minutes après l'injection.
   Chaque cinétique représente une moyenne de 8 animaux.
**Figure 4** : Effet de SAT2 sur la sécrétion d'insuline sur des îlots de Langerhans isolés de pancréas de rat.
**Figure 5** : Effets de SAT2 sur la capture du glucose en présence ou non d'insuline dans la lignée cellulaire de myocytes de rat, les cellules L6.

### EXEMPLES

L'acide chicorique obtenu par le procédé décrit ci-avant (et appelé SAT2 dans nos exemples) est utilisé pour des tests pharmacologiques permettant d'objectiver une activité insulino-stimulante à potentialité amidiabétique. Ces tests ont été effectués *in vitro* sur des cultures de cellules β pancréatiques issues de pancréas de rat et sur des îlots de Langerhans isolés à partir de pancréas de rat ainsi que sur un test in vivo chez le rat.

Dans les exemples rapportés ci-dessous, la concentration d'insuline sécrétée a été évaluée par un système de quantification mis au point par la société Cis Bio International et les inventeurs, utilisant le principe de FRET (9): Insulin Kit.

La glycémie a été dosée par méthode enzymatique en utilisant un kit commercial (Boehringer, Mannheim, Germany). Les résultats ont été soumis à une analyse de variance suivie du test de comparaison multiple.

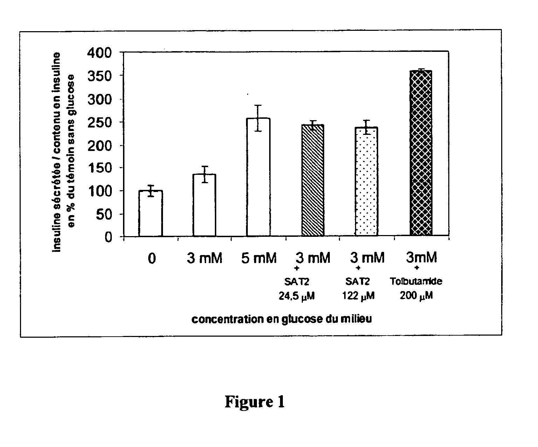

### Exemple 1 : Investigation au niveau de la cellule β en culture (figure 1).

Les cellules β en culture sont des cellules INS 1E issues d'insulinome de rat, mises en culture dans un milieu RPMI 1640 complet. L'intérêt de ces cellules est qu'elles augmentent leur sécrétion d'insuline en fonction de la concentration de glucose dans le milieu (10) et ceci même si ces dernières sont totalement déconnectées de leur environnement physiologique.

Les cellules sont cultivées dans le milieu RPMI à 10% de sérum de veau foetal (SVF) et à 11mM glucose (additionné de pénicilline et streptomycine à 100 µg/mL chacun (antibiotiques), de β-mercaptoéthanol à 50 µM et de sodium pyruvate à 1mM).

Quatre jours après l'ensemencement des cellules sur des plaques 24 puits, le milieu de culture est éliminé et remplacé par du milieu RPMI + 10% de SVF sans glucose. Les cellules sont remises à incuber à 37°C durant 12 heures. Lavées 2 fois avec 1 mL de milieu de Krebs-Ringer sans glucose, les cellules sont mises pendant 90 minutes dans du milieu Krebs-Ringer enrichi en glucose avec ou sans SAT2 (aux concentrations de 10 et 50 µg/mL.)

La figure 1 montre que les cellules INS 1E utilisées sont parfaitement fonctionnelles puisqu'une augmentation de la concentration en glucose dans le milieu de culture de 3 à 5 mM stimule chez ces cellules la sécrétion d'insuline de + 89% (p<0,01).

Conformément à la présente invention, cette expérience démontre que l'addition de 10µg/mL (24,5 µM) de SAT2 dans le milieu contenant 3 mM de glucose (incubation de 90 minutes) permet une augmentation significative de la sécrétion d'insuline (+ 81%, p<0,01). Sur les cellules INS 1E, une telle stimulation de sécrétion d'insuline induite par SAT2 en présence de glucose à 3 mM est équivalente à celle obtenue sur ces cellules lors de l'augmentation de glucose de 3 à 5 mM. Une stimulation de sécrétion d'insuline équivalente est obtenue dans un milieu à 3 mM de glucose mais en présence de 50 µg/mL (122 µM) de SAT2 soit une concentration cinq fois supérieure à l'expérience précédente; ceci suggère que SAT2 atteint très rapidement une stimulation maximale qui est très inférieure à la stimulation obtenue en présence de 200 µM de tolbutamide (sulfonylurée), concentration classiquement utilisée.

Cette stimulation de la sécrétion d'insuline par SAT2 doit permettre d'éviter les hypoglycémies, effet latéral fréquemment observé lors d'un traitement par les sulfonylurées.

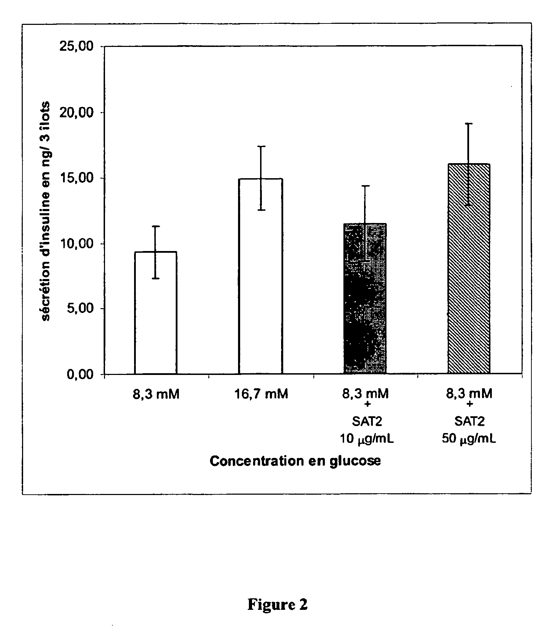

### Exemple 2: Investigation au niveau des îlots de Langerhans isolés à partir de pancréas de rat (figure 2).

Les îlots de Langerhans constituent la partie endocrine du pancréas et contiennent principalement des cellules β qui sécrétent l'insuline. Les îlots de Langerhans de pancréas de rat sont isolés par digestion du pancréas à l'aide de la collagénase selon une méthode adaptée de celle de Lacy et al. (11).

Les îlots ont été séparés des autres éléments du digestat, prélevés sous une loupe binoculaire puis déposés dans des tubes d'incubation. Sur des îlots de rat Wistar normaux incubés dans un tampon Krebs-Ringer avec ou sans SAT2 en présence d'une concentration de glucose de 8,3 mM pendant une heure, nous avons recherché l'effet de différentes concentrations d'acide chicorique sur la sécrétion d'insuline.

Il a été vérifié que les îlots obtenus étaient fonctionnels puisque l'augmentation de la concentration de glucose (de 8,3 à 16,7 mM) induit une stimulation de la sécrétion d'insuline (+ 58%, p<0,05). L'addition d'une concentration de 10 µg/mL d'acide chicorique (SAT 2) lorsque les îlots sont incubés en présence de 8,3mM de glucose déclenche une augmentation de la sécrétion d'insuline (+ 21%).
Une stimulation supérieure est observée lors de l'ajout de SAT 2 à la concentration de 50 µg/mL (+ 68%, p<0,01) pour la même concentration de glucose (8,3 mM).
Cette addition d'acide chicorique provoque une élévation de la sécrétion d'insuline semblable à celle obtenue lors de l'augmentation du glucose seul (à 16,7 mM).

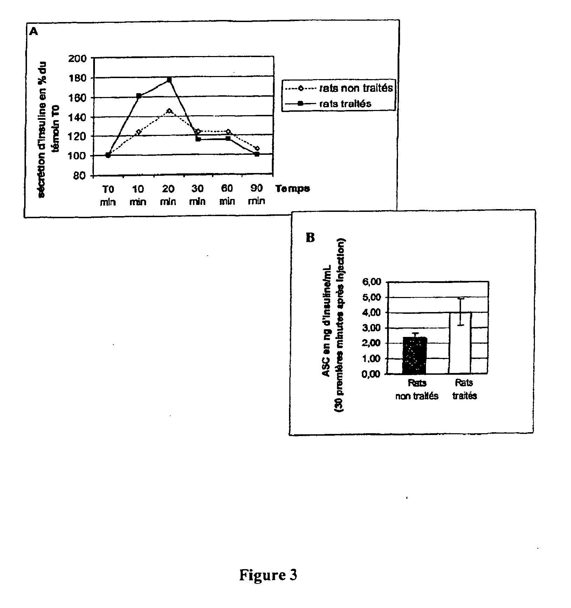

### Exemple 3: Expérimention chez le rat Wistar normal éveillé (figure 3).

L'effet de l'administration d'acide chicorique chez le rat a été testé lors d'une épreuve d'hyperglycémie provoquée (1g glucose/kg de poids vif). Les animaux ont reçu une seule injection intrapéritonéale, soit de glucose seul, soit de glucose plus de l'acide chicorique à la dose de 5mg/kg de poids vif.
Nos résultats démontrent que l'addition d'acide chicorique au glucose a pour effet d'améliorer la réponse insulinique au glucose des animaux. Cette augmentation de l'hyperinsulinémie provoquée par le sucre apparaît lors des 30 premières minutes après l'injection. Le calcul de l'aire sous la courbe (ASC) pendant ces 30 minutes révèle une stimulation de la sécrétion d'insuline clairement supérieure en présence d'acide chicorique (SAT2) (p<0,01).

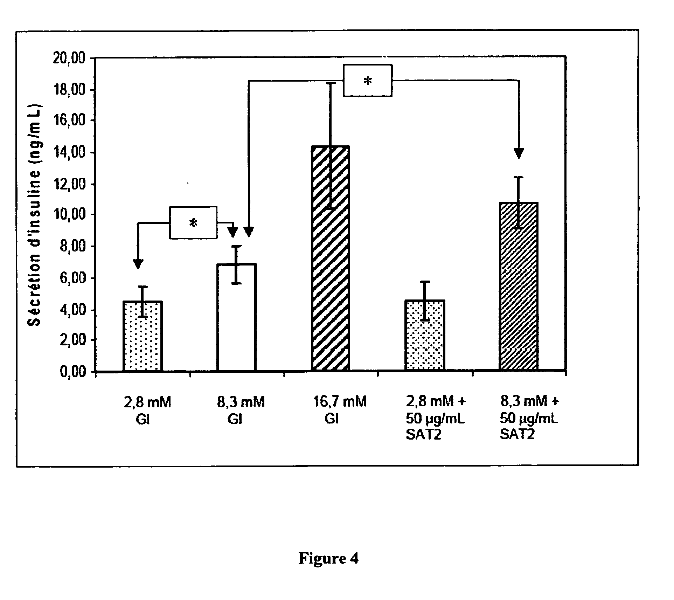

### Exemple 4 : Investigation de l'effet insulino-stimulant de SAT2 en fonction de la concentration de glucose sur des îlots de Langerhans isolés à partir de pancréas de rat (Figure 4)

Après digestion du pancréas par la méthode enzymatique à la collagénase, les îlots ont été séparés des autres éléments du digestat, prélevés sous une loupe binoculaire puis déposés dans des tubes d'incubation. Sur des îlots de rat Wistar normaux incubés dans un tampon Krebs-Ringer en présence d'une concentration de glucose de 2,8, 8,3 et 16,7 mM de glucose pendant une heure. Nous avons recherché l'effet de l'acide chicorique à 50µg/ml sur la sécrétion d'insuline sur des îlots stimulés par 2,8 et 8,3 mM de glucose. Après une heure d'incubation de 3 îlots de Langerhans isolés en présence de différentes concentrations de glucose (Gl) et avec ou sans SAT2 à la concentration de 50 µg/ml. Les résultats obtenus sont rapportés dans la figure 4 et dans le tableau 2 ci-dessous. Les résultats présentés sont la moyenne de 6 points/essai et trois essais indépendants (*, P<0.05)

**Tableau 2**

| | Quantité d'insuline sécrétée en ng (moyenne de 18 essais indépendants) | Ecarts types |
|---|---|---|
| 2,8 mM Gl | 4.45 | 0.95 |
| 8,3 mM Gl | 6.79 | 1.2 |
| 16,7 mM Gl | 14.32 | 3.97 |
| 2,8 mM Gl + 50 µg/mL SAT2 | 4.44 | 1.22 |
| 8,3 mM Gl + 50 µg/mL SAT2 | 10.65 | 1.62 |

A la lecture de ce tableau et de la figure 4, on observe que sous une faible concentration en glucose (2,8 mM), SAT2 ne stimule pas la sécrétion d'insuline des îlots pancréatiques. Lorsque la concentration en glucose est élevée (8,3 mM), une augmentation de la sécrétion d'insuline est observée (+ 52%, P<0,05 par le test de student). La présence de SAT2 sous 8,3 mM de glucose stimule très significativement la sécrétion d'insuline (+ 56%, P<0,05 par le test de student). Donc ces résultats montrent que l'acide chicorique (SAT2) à un effet modulateur glucose dépendant de la sécrétion d'insuline par les îlots pancréatiques.

Cet exemple permet de montrer que l'effet insulino-stimulant de l'acide chicorique est dépendant de la concentration de glucose. Ceci est un résultat important puisque cela démontre que SAT2 (acide chicorique) est capable de stimuler la sécrétion d'insuline sans pour autant provoquer d'hypoglycémie chez le patient comme cela arrive parfois chez les personnes traitées par des sulfonylurées ce qui peut devenir un danger notamment chez les personnes âgées.

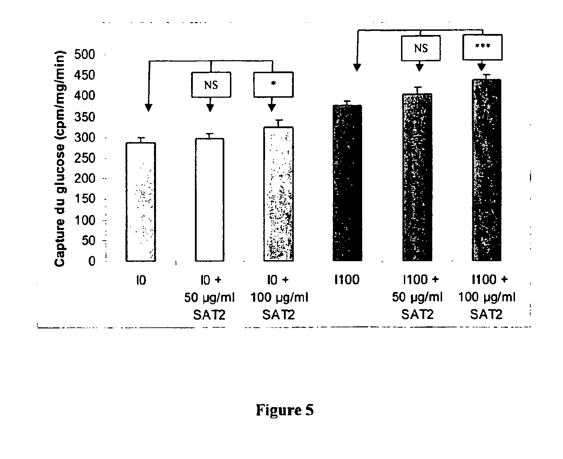

### Exemple 5 : Effets de SAT2 sur la capture du glucose en présence ou non d'insuline dans la lignée cellulaire de myocytes de rat, les cellules L6 (Figure 5).

Pour démontrer l'effet stimulant de l'acide chicorique sur le captage de glucose à une dose stimulante d'insuline, il a été choisi comme modèle cellulaire le myocyte. La lignée L6 (lignée issue du muscle squelettique de *Rattus norvegicus* fournie par ATCC-LCC Promochem) a été utilisée. Le captage de glucose en présence d'insuline avec ou sans SAT2 a été quantifié en utilisant du [3H] deoxyglucose.

La lignée de cellule L6 est cultivée dans le milieu DMEM (4,5 g/l de glucose) complémenté avec 10% de sérum de veau foetal (SVF). Pour les expériences de captage de glucose, les cellules sont ensemencées dans des plaques 12 puits à la densité de 10⁴ cellules /puits. Après trois jours de culture, les cellules sont différentiées dans un milieu DMEM à 2% de SVF durant une semaine. Le jour de l'expérience, les cellules sont privées durant 4h dans un milieu DMEM sans SVF et contenant 0,1% de BSA puis alors elles sont incubées durant 1h dans du tampon Krebs Ringer contenant 1g/l de glucose, contenant 0,100 et 500 nM d'insuline avec ou sans SAT2 à la concentration de 50 ou 100 µg/mL. Les cellules sont alors lavées dans du Krebs-Ringer et incubées en présence de 0,5 µCi de [³H] déoxyglucose dans 1 mL de Krebs-Ringer par puits. Le captage de glucose par les cellules L6 est stoppé par trois lavages dans du PBS froid et les cellules sont alors lysées dans 1 mL d'une solution 0,1N NaOH. La concentration en protéines totales est estimée par la méthode Bradford et la radioactivité est mesurée à l'aide d'un compteur β. Les résultats finaux sont exprimés en cpm/mg/min. L'analyse statistique pour la significativité se fait par le test de student.
Après une semaine de différentiation, les cellules sont stimulées pendant 1 heure en absence (10) et en présence de 100nM d'insuline (I100) combinée à des concentrations croissantes de SAT2 (50 et 100 µg/ml). Les effets sur la capture du glucose sont mesurés en présence de [³H] déoxyglucose pendant 5 minutes. (*, P<0.05 ; ***, P<0.001 ; NS, non significatif).

Résultat : cet exemple montre que SAT2 a un effet insulino-sensibilisateur significatif sur la lignée L6 en culture. En l'absence d'insuline, SAT2 stimule significativement la capture du glucose uniquement à la dose la plus élevée de 100 µg/ml (+ 12.7%, P<0.05). L'insuline à 100 nM augmente la capture du glucose de 30.4% (P<0.001). L'addition de 100 µg/ml de SAT2 permet une amplification de 16.3% de la capture du glucose est observée (P<0.001). La molécule SAT2 est donc capable de stimuler la capture du glucose dans une lignée de myocytes de rat. Une grande partie de cet effet est indépendant de la présence d'insuline (environ 12%). Environ 4% de l'effet est néanmoins dépendant de l'insuline et de ses voies de signalisation.

### Références:

(1) Zimmet P., KG. Alberti, and J. Shaw. Global and societal implications of the diabetes epidemic. Nature 414: 782-787, 2001
(2) Broca C., R. Gross, P. Petit, Y. Sauvaire, M.Manteghetti, M. Tournier, P. Masiello, R. Gomis and G. Ribes. 4-Hydroxyisoleucine : experimental évidence of its insulinotropic and antidiabeti properties. The American Physiological Society E617-623, 1999
(3) Hemmerle H., H.-J. Burger, P. Below, G. Schubert, R. Rippel, P. W. Schindler, E. Paulus and A. W. Herling. Chlorogenic acid and synthetic chlorogenic acid derivatives: novel inhibitors of hepatic glucose-6-phosphate translocase. J Med. Chem, 40, 137-145, 1997
(4) King P.J., G. Ma, W. Mia, Q. Jia, B.R. McDougall, M.G. Reinecke, C.Cornell, J.Kuan, T.R.Kim, W.E.J.R. Robinson, K.Zhu, M.L. Cordeiro, J. Atienza, S.A. Chow, H.H. Lo, J.G. Chung, R. Ejzemberg, MH. Da Silva, L. Pinto, WB. Mors, Y. Jiang, K. Satoh, K. Kusama, S. Watanabe and H. Sakamagi, Structure-activity relationships: analogues of the dicaffeoylquinic and dicaffeoyltartric acides as a potent inhibitors of human immunodeficiency virus type 1 integrase and replication. J. Med. Chemi., 42: 497-509, 1999
(5) Facino R.M., M. Carini, G. Aldini, L. Saibene, P. Pietta and P. Mauri. Echinacoside and caffeoyl conjugates protect collagen from free radical-induced degradation : a potentiel use of Echinacea extracts in the prevention of skin photodomage. Planta Med. 61:510-514, 1995
(6) Dalby-Brown L, H. Barsett, A.K. Landbo, A.S. Meyer and P. Molgaard. Synergistic antioxidative effects of alkamides, caffeic acid derivatives, and polysaccharide fractions from Echinacea purpurea on in vitro oxidation of human low-density lipoproteins. J Agric Food Chem., Nov 30;53(24):9413-23, 2005
(7) Zaho H. and TR. Burke. Facile syntheses of (2R, 3R)-(-)- and (2S, 3S)-(+)-chicoric acids. Synthetic Communications, 28:737-740, 1998
(8) Scarpati ML. and G. Oriente. Chicoric acid (dicaffeoyltartric acid):its isolation from chicory (Cichorium intybus) and synthesis. Tetrahedron, 4:43-48, 1958
(9) Claret E. et al., abstract, congrès The Society for Biomolecular Screening, Sept 11-15, Orlando, Floride, 2004
(10) Sekine N, Fasolato C, Pralong WF, Theler JM, Wollheim CB. Glucose-induced insulin secretion in INS- 1 cells depends on factors present in fetal calf serum and rat islet-conditioned medium. Diabetes 46(9):1424-33, 1997
(11) Lacy P. E., and M. Kostianovsky. Method for the isolation of intact islets of Langerhans from the rat pancreas. Diabetes 16: 35-39, 1967

## Revendications

1. Composition pour utilisation dans la prévention ou le traitement chez un sujet de l'insulino-résistance ou de l'hypo-insulinémie et/ou des pathologies associées choisies dans le groupe constitué par le diabète, les dyslipidémies, les hyperlipidémies, l'hypertriglycéridémie, le syndrome métabolique et l'obésité, **caractérisée en ce qu'**elle comprend de l'acide chicorique, ladite composition ayant une activité insulino-stimulante dépendante de la concentration en glucose dans le sang dudit sujet.

2. Composition pour utilisation selon la revendication 1, **caractérisée en ce que** la composition est insulino-stimulante et qu'elle est destinée à la prévention ou au traitement de l'hypo-insulinémie.

3. Composition pour utilisation selon la revendication 1 ou 2, **caractérisée en ce que** des pathologies liées à l'insulino-résistance ou à l'hypoinsulinémie sont choisies dans un groupe comprenant le diabète, le syndrome métabolique et l'obésité.

4. Composition pour utilisation selon la revendication 2, **caractérisée en ce que** la pathologie est le diabète.

5. Composition pour utilisation selon la revendication 3, **caractérisée en ce que** le diabète est un diabète non insulino dépendant ou insulino dépendant.

6. Composition pour utilisation selon l'une quelconque des revendications 1 à 5, **caractérisée en ce qu'**elle est insulino-stimulante chez un sujet présentant une concentration sanguine en glucose supérieure à 1 g/l.

7. Composition pour utilisation selon l'une quelconque des revendications 1 à 6, **caractérisée en ce que** l'acide chicorique est d'origine végétale ou synthétique.

8. Composition pour utilisation selon la revendication 7, **caractérisée en ce que** l'acide chicorique est obtenu à partir de plantes appartenant à la famille des Asteraceae, des Lamiaceae, des Fabaceae, d'Equisetaceae ou de Potamogetonaceae.

9. Composition pour utilisation selon l'une quelconque des revendications 1 à 8, **caractérisée en ce que** l'acide chicorique est obtenu à partir de chicorées sauvages ou cultivées.

10. Composition pour utilisation selon l'une quelconque des revendications 1 à 9, **caractérisée en ce que** l'acide chicorique est sous la forme d'un des isomères acide dicaféoyl (+), (-) ou mésotartrique.

11. Composition pour utilisation selon l'une quelconque des revendications 1 à 10, **caractérisée en ce qu'**elle comprend l'administration au sujet de 5 à 30 mg/Kg d'acide chicorique par prise.

12. Composition pour utilisation selon l'une des revendications 1 à 11, pour stimuler la sécrétion d'insuline et pour améliorer la tolérance au glucose.

## Claims

1. A composition for use in the prevention or treatment in a subject of insulin resistance or hypoinsulinemia and/or associated pathologies selected from the group consisting of diabetes, dyslipidemias, hyperlipidemia, hypertriglyceridemia, metabolic syndrome and obesity, **characterized in that** it comprises chicoric acid, said composition having insulin-stimulating activity dependent on the concentration of glucose in the blood of said subject.

2. The composition for use according to claim 1, **characterized in that** the composition is insulin-stimulating and is intended for the prevention or treatment of hypoinsulinemia.

3. The composition for use according to claim 1 or claim 2, **characterized in that** the pathologies related to insulin resistance or hypoinsulinemia are chosen from a group comprising diabetes, metabolic syndrome and obesity.

4. The composition for use according to claim 2, **characterized in that** the pathology is diabetes.

5. The composition for use according to claim 3, **characterized in that** the diabetes is non-insulin-dependent or insulin-dependent diabetes.

6. The composition for use according to any one of claims 1 to 5, **characterized in that** it is insulin-stimulating in a subject with a blood glucose concentration greater than 1 g/1.

7. The composition for use according to any one of claims 1 to 6, **characterized in that** the chicoric acid is of plant or synthetic origin.

8. The composition for use according to claim 7, **characterized in that** the chicoric acid is obtained from plants belonging to the family of Asteraceae, Lamiaceae, Fabaceae, Equisetaceae or Potamogetonaceae.

9. The composition for use according to any one of claims 1 to 8, **characterized in that** the chicoric acid is obtained from wild or cultivated chicories.

10. The composition for use according to any one of claims 1 to 9, **characterized in that** the chicoric acid is in the form of a (+), (-) or meso isomer of dicaffeoyl-tartaric acid.

11. The composition for use according to any one of claims 1 to 10, **characterized in that** it comprises the administration in the subject of 5 to 30 mg/kg of chicoric acid per dose.

12. The composition for use according to any one of claims 1 to 11, to stimulate insulin secretion and to improve glucose tolerance.

## Patentansprüche

1. Zusammensetzung zur Verwendung bei der Verhütung oder der Behandlung von Insulinresistenz oder von Hypoinsulinämie und/oder von damit verbundenen Pathologien, ausgewählt aus der Gruppe bestehend aus Diabetes, Dyslipidämien, Hyperlipidämie, Hypertriglyzeridämie, dem metabolischen Syndrom und Obesität, bei einem Individuum, **dadurch gekennzeichnet, dass** sie Chicorsäure umfasst, wobei die Zusammensetzung eine von der Glucosekonzentration im Blut des Individuums abhängige Insulin-stimulierende Aktivität aufweist.

2. Zusammensetzung zur Verwendung nach Anspruch 1, **dadurch gekennzeichnet, dass** die Zusammensetzung Insulin-stimulierend ist und dass sie für die Verhütung oder die Behandlung von Hypoinsulinämie bestimmt ist.

3. Zusammensetzung zur Verwendung nach Anspruch 1 oder 2, **dadurch gekennzeichnet, dass** mit Insulinresistenz oder Hypoinsulinämie verbundene Pathologien aus einer Gruppe, umfassend Diabetes, das metabolische Syndrom und Obesität, ausgewählt sind.

4. Zusammensetzung zur Verwendung nach Anspruch 2, **dadurch gekennzeichnet, dass** die Pathologie Diabetes ist.

5. Zusammensetzung zur Verwendung nach Anspruch 3, **dadurch gekennzeichnet, dass** der Diabetes ein nicht-insulinabhängiger oder insulinabhängiger Diabetes ist.

6. Zusammensetzung zur Verwendung nach einem der Ansprüche 1 bis 5, **dadurch gekennzeichnet, dass** sie bei einem Individuum, welches einen Blutzuckerspiegel über 1 g/l aufweist, Insulin-stimulierend ist.

7. Zusammensetzung zur Verwendung nach einem der Ansprüche 1 bis 6, **dadurch gekennzeichnet, dass** die Chicorsäure aus pflanzlichen oder synthetischen Ursprungs ist.

8. Zusammensetzung zur Verwendung nach Anspruch 7, **dadurch gekennzeichnet, dass** die Chicorsäure ausgehend von Pflanzen, welche zu der Familie der Asteraceae, der Lamiaceae, der Fabaceae, der Equisetaceae oder der Potamogetonaceae gehören, erhalten wird.

9. Zusammensetzung zur Verwendung nach einem der Ansprüche 1 bis 8, **dadurch gekennzeichnet, dass** die Chicorsäure ausgehend von wilden oder kultivierten Zichorien erhalten wird.

10. Zusammensetzung zur Verwendung nach einem der Ansprüche 1 bis 9, **dadurch gekennzeichnet, dass** die Chicorsäure in Form von einem der (+)-, (-)- oder meso-Dicaffeoylweinsäure-Isomeren vorliegt.

11. Zusammensetzung zur Verwendung nach einem der Ansprüche 1 bis 10, **dadurch gekennzeichnet, dass** sie die Verabreichung von 5 bis 30 mg/kg Chicorsäure pro Einnahme an das Individuum umfasst.

12. Zusammensetzung zur Verwendung nach einem der Ansprüche 1 bis 11, um die Insulinsekretion zu stimulieren und um die Toleranz gegenüber Glucose zu verbessern.
